(19) Europäisches Patentamt
European Patent Office
Office européen des brevets

(11) Veröffentlichungsnummer: **0 166 230**
**B1**

(12) # EUROPÄISCHE PATENTSCHRIFT

(45) Veröffentlichungstag der Patentschrift: **06.04.88**

(51) Int. Cl.⁴: **C 07 D 241/42,** G 03 G 5/06, G 03 G 5/04

(21) Anmeldenummer: **85106442.8**

(22) Anmeldetag: **24.05.85**

(54) **2,3-Bis(dialkylaminophenyl)chinoxaline und ihre Verwendung in elektrophotographischen Aufzeichnungsmaterialien.**

(30) Priorität: **29.05.84 DE 3420039**

(43) Veröffentlichungstag der Anmeldung: **02.01.86 Patentblatt 86/1**

(45) Bekanntmachung des Hinweises auf die Patenterteilung: **06.04.88 Patentblatt 88/14**

(84) Benannte Vertragsstaaten: **DE FR GB**

(56) Entgegenhaltungen:
**CH-A-599 569**
**DE-B-1 254 469**
**US-A-4 125 725**

**CHEMICAL ABSTRACTS, vol. 87, no. 18, 31. Oktober 1977, Columbus, Ohio, USA, A. GERLICZ, J. KRASKA, L. WOJCIECHOWSKI, "Azo pigments, derivatives of quinoxaline", S. 67, Zusammenfassung Nr. 137 307w**
**CHEMICAL ABSTRACTS, vol. 90, no. 8, 19. Februar 1979, Columbus, Ohio, USA, J. KRASKA, A. GERLICZ, "Disazo condensation pigments, derivatives of quinoxaline", S. 75, Zusammenfassung Nr. 56 315g**
**CHEMICAL ABSTRACTS, vol. 101, no. 5, 30. Juli 1984, Columbus, Ohio, USA, F. ROUBINEK, V. BYDZOVSKY, Z. BUDESINSKY, "Substituted 5- and 6-quinoxalinecarboxylic acids and their tuberculostatic activity", S. 508, Zusammenfassung Nr. 38 427m**
**CHEMICAL ABSTRACTS, vol. 96, no. 23, 7. Juni 1982, Columbus, Ohio, USA, K.V. SOLODOVA, L.A. SURKINA; "2-(4'-Aminophenyl)quinoxaline", S. 678, Zusammenfassung Nr. 199 729e**
**CHEMICAL ABSTRACTS, vol. 85, no. 22, 29. November 1976, Columbus, Ohio, USA, AMERICAN HOECHST CORP., "Photopolymer multicolor proofs", S. 673, Zusammenfassung Nr. 169 706a**
**CHEMICAL ABSTRACTS, vol. 89, no. 3, 17. Juli 1978, Columbus, Ohio, USA, A.A. JARRAR "Photochemistry of some quinoxaline 1,4-dioxides", S. 649, Zusammenfassung Nr. 24 276h**
**CHEMICAL ABSTRACTS, vol. 87, no. 19, 7. November 1977, Columbus, Ohio, USA, A. GERLICZ, J. KRASKA, L. WOJCIECHOWSKI, "Quinoxaline derivatives", S. 603, Zusammenfassung Nr. 152 269d**

(73) Patentinhaber: **HOECHST AKTIENGESELLSCHAFT, Postfach 80 03 20, D-6230 Frankfurt am Main 80 (DE)**

(72) Erfinder: **Pawlowski, Georg, Dr. Dipl.- Chem., Blücherstrasse 48, D-6200 Wiesbaden (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 166 230 B1

LIBER, STOCKHOLM 1988

**Beschreibung**

Die Erfindung betrifft neue 2,3-Bis(dialkyl-aminophenyl) chinoxaline und ihre Verwendung als photoleitfähige Substanzen in elektrophotographischen Aufzeichnungsmaterialien, insbesondere für den Flachdruck oder die Resisttechnik.

Die Verwendung von Chinoxalinderivaten als Photoleiter in elektrophotographischen Aufzeichnungsmaterialien ist bereits bekannt (DE-PS 12 54 469 entsprechend GB-PS 1,062,935 und GB-PS 1,004,461). Die dort beschriebenen Chinoxalinderivate zeigen jedoch keine hohen Ansprüchen genügenden Lichtempfindlichkeiten. So sind beispielsweise derartige Photoleiter enthaltende elektrophotographische Aufzeichnungsmaterialien für eine laserbelichtung infolge mangelnder Empfindlichkeit nicht besonders geeignet, da kein ausreichender Ladungskontrast zwischen belichteten und nicht belichteten Schichtteilen erhalten wird. Auch bei der Verwendung herkömmlicher Lichtquellen erweisen sich diese Aufzeichnungsmaterialien als weniger gut geeignet, da sie selbst bei lang anhaltender, intensiver Belichtung eine unerwünscht große Restladung aufweisen. Dies führt dazu, daß auch belichtete Schichtbereiche bei Entwicklung Tonermaterial annehmen und zu einem wenig randscharfen oder schlecht aufgelösten sichtbaren Bild führen.

Es bestand daher die Aufgabe, für elektrophotographische Aufzeichnungsmaterialien guter Lichtempfindlichkeit und intensiven Kontrasts wirksamere Photoleiter anzugeben.

Es wurde überraschend gefunden, daß sich hochlichtempfindliche elektrophotographische Aufzeichnungsmaterialien mit geringem Restpotential und großem Kontrast dann erhalten lassen, wenn man solche Chinoxalinderivate als photoleitfähige Substanzen verwendet, die im carbocyclischen Ring eine oder mehrere Alkyl- oder Alkoxygruppen tragen, ohne daß dadurch andere für den elektrophotographischen Prozeß relevante Eigenschaften wie Dunkelentladung oder drucktechnische Eigenschaften nachteilig beeinflußt werden.

Gegenstand der Erfindung sind 2,3-Bis(dialkylaminophenyl)chinoxaline der allgemeinen Formel

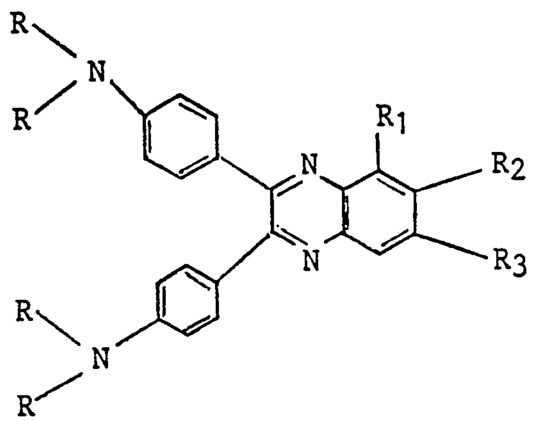

in welcher R Alkyl mit bis zu 4 Kohlenstoffatomen,

$R_1$ Wasserstoff oder Alkyl mit bis zu 4 Kohlenstoffatomen,

$R_2$ gleich oder verschieden ist von $R_1$ und Alkyl oder Alkoxy mit bis zu 4 Kohlenstoffatomen und

$R_3$ gleich oder verschieden ist von $R_2$ und Wasserstoff, Alkyl oder Alkoxy mit bis zu 4 Kohlenstoffatomen bedeuten.

Gegenstand der Erfindung ist auch die Verwendung der erfindungsgemäßen 2,3-Bis(dialkylaminophenyl)chinoxaline der allgemeinen Formel als photoleitfähige Substanzen in elektrophotographischen Aufzeichnungsmaterialien, insbesondere für Flachdruckformen oder Resistmaterialien, wobei die erfindungsgemäßen Chinoxalinderivate gegebenenfalls auch in Kombination mit anderen organischen oder anorganischen Photoleitertypen und üblichen Zusätzen eingesetzt werden können.

Bevorzugt sind solche 2,3-Bis(dialkylaminophenyl)chinoxaline der allgemeinen Formel, in der R Methyl oder Ethyl, $R_1$ Wasserstoff oder Methyl, $R_2$ Methyl, Ethyl, Methoxy oder Ethoxy und $R_3$ Wasserstoff, Methyl oder Methoxy bedeuten.

Ganz besonders bevorzugt sind solche 2,3-Bis(dialkylaminophenyl)chinoxaline der allgemeinen Formel, in der R Methyl oder Ethyl, $R_1$ und $R_3$ Wasserstoff oder Methyl und $R_2$ Methyl, Methoxy oder Ethoxybedeuten, wie beispielsweise

2,3-Bis(4'-dimethylaminophenyl)-6-methyl-chinoxalin, Fp.: 163 bis 164° C;

2,3-Bis(4'-dimethylaminophenyl)-6-methoxy-chinoxalin, Fp.: 194 bis 195° C;

2,3-Bi5(4'-dimethylaminophenyl)-6-ethoxy-chinoxalin, Fp.: 179 bis 181° C;

2,3-Bi5(4'-dimethylaminophenyl)-5,6-dimethyl-chinoxalin, Fp. 212 bis 213° C;

2,3-Bi5(4'-dimethylaminophenyl)-6,7-dimethyl-chinoxalin, Fp.: 196 bis 197° C;

2,3-Bis(4'-diethylaminophenyl)-6-methyl-chinoxalin, Fp.: 142 bis 143,5° C;

2,3-Bis(4'-diethylaminophenyl)-6-methoxy-chinoxalin, Fp.: 129 bis 130,5°C;
2,3-Bis(4'-diethylaminophenyl)-6-ethoxy-chinoxalin, Fp.: 112 bis 113°C;
2,3-Bis(4'-diethylaminophenyl)-5,6-dimethyl-chinoxalin, Fp.: 142 bis 143 °C;
2,3-Bis(4' -diethylaminophenyl)-6,7-dimethyl-chinoxalin, Fp.: 126 bis 128°C.

Die erfindungsgemäßen Chinoxaline sind neu. Ihre Herstellung ist als im Prinzip bekannt anzusehen und kann beispielsweise durch Umsetzung des entsprechenden Bis-dialkylaminobenzils mit dem entsprechenden o-Phenylendiamin in einem polar protischen Lösungsmittel gegebenenfalls unter Zusatz eines sauren Katalysators [vgl. W. Bost und E.E. Towell, J. Amer. Chem. Soc., 70, 903 (1948)] erfolgen.

Die entsprechend substituierten Benzile wie z.B. 4,4'-Bis-dimethylaminobenzil, 4,4'-Bis-diethylaminobenzil, 4,4'-Bis-dipropylaminobenzil usw. sind nach bekannten Literaturvorschriften erhältlich [C. Tüzün, M. Ogliaruso und E.I. Becker, Org. Synth. Coll. Vol. V, S. 111 (1973)].

Zahlreiche der o-Phenylenderivate sind im Handel erhältlich oder nach bekannten Literaturvorschriften herstellbar. So können 3,4-Diaminotoluol, 3,4-Diaminoanisol, 3,4-Diaminophenetol, 3,4-Diamino-o-xylol, 4,5-Diamino-o-xylol oder 4,5-Diaminoveratrol [vgl. z.B. J. Ehrlich und T.M. Bogert, J. Org. Chem., 12, 522 (1947)] Verwendung finden. Die Bildung der erfindungsgemäßen Chinoxaline kann in ethanolischer Lösung unter Chlorwasserstoff-Katalyse oder in essigsaurer Lösung ohne zusätzlichen Katalysator vorgenommen werden. Die Umsetzung erfolgt unter Rückflußbedingungen und verläuft im allgemeinen innerhalb von drei Stunden mit fast quantitativer Ausbeute. Die intensiv gelb gefärbten Produkte werden durch Ausfällung in Wasser, welches gegebenenfalls alkalisch eingestellt ist, isoliert. Ihre Reinigung kann durch Umkristallisation aus einem Alkohol/Wasser-Gemisch erfolgen.

Die erfindungsgemäßen Chinoxaline der allgemeinen Formel zeigen eine unerwartet viel größere Lichtempfindlichkeit als die bekannten und in der DE-PS 12 54 469 und der GB-PS 1,004,461 aufgeführten Verbindungen. Es war überraschend, daß zur Erreichung hoher Lichtempfindlichkeiten des Photoleiters das Zusammenwirken mindestens zweier Dialkylaminophenylgruppen in 2- und 3-Stellung und mindestens einer elektronenspendenden Alkyl- oder Alkoxylgruppe in der 5-, 6- oder 7-Stellung des Chinoxalinringes notwendig ist.

Es haben sich solche Chinoxaline der allgemeinen Formel als besonders vorteilhaft erwiesen, in der $R_1$, $R_2$ und $R_3$ gleich oder verschieden sind und Wasserstoff, Methyl, Methoxy oder Ethoxy bedeuten, wobei höchstens zwei der Reste $R_1$, $R_2$ und $R_3$ gleichzeitig Wasserstoff, Methyl, Methoxy oder Ethoxy darstellen.

Es war auch überraschend, daß sich bei den erfindungsgemäßen Chinoxalinen andere, für den elektrophotographischen Prozeß sowie die für druck- und kopiertechnische Eigenschaften wichtigen Parameter nicht verschlechtern, sondern im Gegenteil ebenfalls höheren Ansprüchen genügende Eigenschaften aufweisen.

So zeigen die photoleitfähigen Schichten mit Chinoxalinderivaten der allgemeinen Formel beispielsweise nach Aufladung und Lagerung im Dunkeln nur einen sehr geringen Spannungsabfall. Dies ist überraschend, da für zahlreiche photoleitfähige Substanzen bekannt ist, daß eine Überladung eines Moleküls mit elektrophotographisch aktiven Substituenten wie z.B. einer Dialkylaminogruppe oder ähnlichen elektronenspendenden Substituenten in vielen Fällen zu einer hohen Dunkelentladung führt.

Gleichzeitig findet bei der Belichtung des elektrophotographischen Aufzeichnungsmaterials nach der Erfindung eine rasche und sehr vollständige Entladung statt, so daß ein ungewöhnlich hoher Spannungskontrast zwischen belichteten und unbelichteten Schichtteilen entsteht und bei der Entwicklung mit feinteiliger Tonersubstanz einwandfreie, randscharfe und hochaufgelöste, auch feinste Bildelemente wiedergebende Abbildungen der Vorlage erhalten werden.

Diese Eigenschaften lassen die erfindungsgemäßen Chinoxaline sowie die sie enthaltenden Aufzeichnungsmaterialien auch bei Anwendung von Flüssigentwicklung oder, bei entsprechender Sensibilisierung, für Belichtungen mittels Laserstrahl interessant erscheinen.

Als weitere Vorteile der erfindungsgemäßen Chinoxalinderivate der allgemeinen Formel enthaltenden Aufzeichnungsmaterialien seien ihre rasche Aufladbarkeit, hohe Aufladungskapazität und ihre hervorragende Verträglichkeit mit Polymeren der verschiedensten chemischen Zusammensetzungen erwähnt, wobei infolge der geringen Kristallisationstendenz der erfindungsgemäßen Chinoxaline bei Bedarf hohe Anteile derselben bis zu 95 % keinerlei Kristallisationstendenz zeigen.

Die erfindungsgemäßen Chinoxaline sind darüber hinaus in hohem Maße verträglich mit anderen organischen oder anorganischen Photoleitertypen, so daß neben Mischungen der Chinoxaline untereinander auch Mischungen mit anderen Verbindungen eingesetzt werden können, die zum Teil hervorragende elektrophotographische Eigenschaften besitzen.

Elektrophotographische Aufzeichnungsmaterialien mit den erfindungsgemäßen Chinoxalinen lassen sich sowohl negativ als auch positiv aufladen.

Schließlich haben toxikologische Untersuchungen ergeben, daß die erfindungsgemäßen Chinoxaline physiologisch unbedenklich sind, was insbesondere im Hinblick auf Umweltbelastungen von großer Bedeutung ist.

Die erfindungsgemäßen Chinoxaline sind in üblichen Lösungsmitteln sehr gut löslich. Daher bestehen von Seiten der Photoleiter keinerlei Beschränkungen bezüglich der bei der Beschichtung verwendeten Lösungsmittel oder nachfolgend eingesetzten Verarbeitungschemikalien, so daß deren Auswahl in weiten Bereichen hinsichtlich ihrer Umweltfreundlichkeit erfolgen kann.

Wenngleich die erfindungsgemäßen Chinoxaline für sich filmbildend sind, ist es doch zweckmäßig, diese mit

organischen Polymerisaten zur Erhaltung mechanisch widerstandsfähiger Aufzeichnungsmaterialien zu mischen.

Für die Auswahl des polymeren Bindemittels sind neben den Filmbildungseigenschaften die Alkalilöslichkeit, die elektrischen Eigenschaften sowie die Haftungseigenschaften auf dem elektrisch leitenden Schichtträger, Druckdauerhaftigkeit und schließlich die physiologische Unbedenklichkeit entscheidend. Besonders geeignet sind solche Bindemittel, die in einem wäßrigen oder alkoholischen Lösungsmittel unter Zusatz von Säure oder bevorzugt von Alkali löslich sind.

Vorteilhafterweise werden hochmolekulare Bindemittel mit ausreichender Alkalilöslichkeit verwendet. Die Alkalilöslichkeit läßt sich durch Einbau gewisser Gruppen wie beispielsweise Säureanhydridgruppen, Carboxylgruppen, phenolische und aliphatische Hydroxygruppen, Sulfonsäure-, Sulfonamid- oder Sulfonimidgruppen oder durch elektronenziehende Gruppen aktivierte Urethangruppen erreichen.

Für die Herstellung von lichtempfindlichen Schichten für den Drucksektor sind Copolymerisate mit Säureanhydridgruppen, teilveresterten Säureanhydridgruppen, Carboxylgruppen und Phenolharzen besonders geeignet, da derartige Mischungen hervorragende elektrophotographische Eigenschaften und sehr gute drucktechnische Eigenschaften miteinander verbinden.

Insbesondere seien folgende Polymerisate erwähnt: Copolymerisate aus Styrol oder substituierten Styrolen mit Maleinsäureanhydrid, Copolymerisate aus Styrol oder substituiertem Styrol mit teilverestertem Maleinsäureanhydrid, Copolymerisate aus Acrylsäure, Methacrylsäure und Acrylsäureestern sowie Umsetzungsprodukte aus freie Hydroxylgruppen enthaltenden Polyvinylacetalen und Sulfonylisocyanaten.

Werden Phenolharze als Bindemittel eingesetzt, so werden Homo- oder Copolymerisate des Hydroxystyrols oder Novolakharze bevorzugt, wobei letztere beispielsweise durch Kondensation von Phenol oder Kresol mit Formaldehyd erhältlich sind.

Der Anteil des Bindemittels kann infolge der filmbildenden Eigenschaften der erfindungsgemäßen Chinoxaline in weiten Grenzen variiert werden, ohne dar es zu einer Kristallisation oder einem Ausschwitzen der photoleitfähigen Substanz kommt. Bevorzugt sind Ausführungen, in denen das Gewichtsverhältnis Polymerisat zu Photoleiter zwischen 1 zu 20 und 4 zu 1 variiert. Die besten Ergebnisse werden bei einem Verhältnis zwischen 1 zu 2 und 2 zu 1 erreicht. Der Photoleiteranteil beträgt hiernach zwischen 20 und 95 Gewichtsprozent, bezogen auf die Photoleiterschicht.

Wenngleich das elektrophotographische Aufzeichnungsmaterial nach der Erfindung von sich aus photoleitfähig ist, läßt sich seine Lichtempfindlichkeit gegenüber sichtbarem Licht, insbesondere in speziell interessanten spektralen Bereichen, durch den Zusatz von sensibilisierenden Farbstoffen erheblich erhöhen.

Als geeignete Sensibilisierungsfarbstoffe seien Triphenylmethanfarbstoffe wie Malachitgrün (C.I. 42000), Brillantgrün (C.I. 42040), Kristallviolett (C.I. 42555) und dergleichen, Thiazinfarbstoffe wie Methylenblau (C.I. 52015), Methylengrün (C.I. 52020) und dergleichen, Oxazinfarbstoffe wie Caprylblau (C.I. 48035) und dergleichen, Astrazonfarbstoffe wie Astrazongelb 3GL (C.I. 48035), Astrazonorange R (C.I. 48040), Astrazonrot (C.I. 48020) und dergleichen, Cyaninfarbstoffe wie Aizen Astra Phloxin FF (C.I. 48070) und dergleichen, Xanthenfarbstoffe wie Rhodamin FB (C.I. 45170) und dergleichen und Pyrylium- bzw. Benzopyryliumfarbstoffe sowie geeignete Kombinationen dieser Farbstoffe genannt.

Weiterhin können in diesen Matrizes lösliche Indigoid-, Chinacridon- oder Azofarbstoffe eingesetzt werden.

Besonders bevorzugt werden als Sensibilisierungsfarbstoffe dabei Astrazonorange R (C.I. 48040), Rhodamin FB (C.I. 45170) und Brillantgrün (C.I. 42040) allein oder in Mischung eingesetzt.

Die der photoleitfähigen Schicht zuzusetzende Menge an Sensibilisierungsfarbstoff kann in Grenzen von etwa 0,01 bis 30 % schwanken. Bevorzugt werden zwischen 1 und 50 %, bezogen auf eingesetzten Photoleiter, verwendet.

Als geeignete Schichtträger für das elektrophotographische Aufzeichnungsmaterial nach der Erfindung werden elektrisch leitende Folien oder Bleche mit hydrophilen Oberflächen verwendet, beispielsweise elektrisch leitfähiges Papier, Aluminium- oder Zinkfolien und -bleche, Mehrmetallbleche wie Kupfer-Aluminiumbleche, Chrom-Kupferbleche und dergleichen oder metallbedampfte oder metallisierte Kunststoffolien.

Besonders bevorzugt sind als Schichtträger Aluminiumbleche oder -folien, die zwecks Hydrophilierung ihrer Oberfläche einer geeigneten Vorbehandlung unterzogen werden.

Zu diesem Zweck wird das walzblanke Aluminiumblech oberflächlich durch mechanisches Bürsten oder elektrochemisch aufgerauht, gegebenenfalls sauer oder alkalisch gebeizt, in einer geeigneten Säure anodisiert und schließlich durch Behandeln mit Silikat oder Polyvinylphosphonsäure hydrophiliert. Neben der Hydrophilierung wird durch diese Maßnahmen das Auftreten schädlicher Reaktionen mit der auf der Oberfläche des Schichtträgers auszubildenden elektrophotographischen Schicht verhindert.

Falls erwünscht, kann zwischen die Oberfläche des Schichtträgers und die photoleitfähige Beschichtung eine Zwischenschicht gebracht werden, die beispielsweise zu einer Haftverbesserung des photoleitfähigen Materials führt. Ebenso kann über der photoleitfähigen Schicht eine Deckschicht ausgebildet sein, die bei der Entschichtung abgelöst werden kann, wodurch gegebenenfalls die elektrostatischen Eigenschaften der photoleitfähigen Schicht verbessert werden und die Schicht selbst geschützt wird vor mechanischem Angriff.

Die Herstellung des elektrophotographischen Aufzeichnungsmaterials erfolgt durch Lösen des Bindemittels, des Photoleiters, der Sensibilisatoren und sonstiger Zusätze in einem geeigneten Lösungsmittel oder einem Lösungsmittelgemisch.

Eine derart erhaltene homogene Lösung wird auf den Schichtträger gebracht und getrocknet. Das

Schichtgewicht der getrockneten Schicht beträgt zwischen 1 $g/m^2$ und 200 $g/m^2$, vorzugsweise zwischen 2 $g/m^2$ und 15 $g/m^2$.

Geeignete Lösungsmittel zur Herstellung einer homogenen Lösung der vorstehend beschriebenen Komponenten umfassen Alkohole, Ketone, Etheralkohole, Ether und Ester, wobei die Siedepunkte der verwendeten Lösungsmittel bei Normaldruck vorzugsweise unterhalb 150° liegen und diese physiologisch unbedenklich sind.

Wird das erfindungsgemäße elektrophotographische Aufzeichnungsmaterial zur Herstellung von Flachdruckformen verwendet, so wird die beschichtete Aluminiumfolie mittels Coronaentladung aufgeladen und einer Belichtung, beispielsweise einer Rasterbelichtung, einer Kontaktbelichtung oder einer Laserbelichtung ausgesetzt. Anschließend wird das latente Bild auf der Schicht mit einem feinteiligen Entwickler behandelt, das erhaltene Tonerbild fixiert und durch nachfolgendes Entschichten in die Druckvorlage überführt, wobei die keinen Toner tragenden Schichtbereiche entfernt werden.

Die erhaltene Druckvorlage ist an den Stellen, an denen die Schichtträgeroberfläche freigelegt wurde, hydrophil, während sie an den Stellen, an denen Toner auf der lichtempfindlichen Schicht haftet, oleophil ist. Sie ist damit in einem druckfertigen Zustand. Entsprechendes gilt für die Herstellung gedruckter Schaltungen.

Das elektrophotographische Aufzeichnungsmaterial nach der Erfindung kann durch geeignete Zusätze wie Chinondiazidverbindungen modifiziert werden, wodurch nach einer Gesamtbelichtung nach der Ausbildung des Tonerbildes eine Verbesserung der Löslichkeit der Nichtbildstellen bewirkt werden kann.

Als weitere übliche Zusätze können in der photoleitfähigen Schicht Verlaufmittel, Weichmacher oder Haftvermittler eingesetzt werden.

Das elektrophotographische Aufzeichnungsmaterial nach der Erfindung wird bevorzugt für die Herstellung von Druckplatten mit großer Lichtempfindlichkeit, guter Auflösung, guter Druckfarbenaufnahmeeigenschaften und hoher Auflage verwendet. Weiterhin läßt sich das vorliegende Aufzeichnungsmaterial für ätzbare Resiste vorteilhaft verwenden.

Anhand folgender Beispiele wird die Erfindung näher erläutert, ohne daß die Erfindung auf diese begrenzt ist. In der beigefügten Formeltabelle 1 werden bevorzugt genannte Verbindungen I bis IV der erfindungsgemäßen Chinoxaline aufgeführt, die mit einigen (V bis VIII), in Formeltabelle 2 zusammengestellten Chinoxalinen des genannten Standes der Technik verglichen werden (DE-PS 12 54 469 GB-PS 1,004,461).

Die Verbindungen werden durch folgende Daten charakterisiert:

| Verbindung nach Formel | Fp. (°C) | | Analysenergebnisse (in %) C | H | N | Cl |
|---|---|---|---|---|---|---|
| I | 142-143,5 | berechnet: | 79,4 | 7,8 | 12,8 | |
| | | gefunden: | 79,7 | 7,8 | 12,8 | |
| II | 163-164 | berechnet: | 78,5 | 6,9 | 14,7 | |
| | | gefunden: | 78,4 | 6,9 | 14,7 | |
| III | 112-113 | berechnet: | 76,9 | 7,7 | 11,9 | |
| | | gefunden: | 77,1 | 7,8 | 12,0 | |
| IV | 196-197 | berechnet: | 78,7 | 7,1 | 14,2 | |
| | | gefunden: | 78,8 | 7,0 | 14,1 | |
| V | 185-186 | berechnet: | 73,4 | 5,0 | 11,7 | 9,9 |
| | | gefunden: | 73,8 | 5,1 | 11,5 | 9,6 |
| VI | 185-189 | berechnet: | 77,7 | 5,9 | 11,8 | |
| (Isomerengemisch) | | gefunden: | 77,4 | 5,9 | 11,8 | |
| VII | 123 | berechnet: | 81,2 | 5,9 | 12,9 | |
| | | gefunden: | 81,0 | 5,9 | 12,7 | |
| VIII | 140-155 | berechnet: | 81,4 | 6,2 | 12,4 | |
| (Isomerengemisch) | | gefunden: | 81,1 | 6,4 | 12,6 | |

**Beispiel 1**

Eine Beschichtungslösung, bestehend aus

30,0 g eines Copolymerisats von Styrol und Maleinsäureanhydrid mit einem mittleren Molekulargewicht von 80.000,

23,0 g 2,3-Bis(4-diethylaminophenyl)-6-ethoxy-chinoxalin /Verbindung III),

0,1 g Rhodamin FB (C.I. 45170),

0,6 g Astrazonorange R (C.I. 48040) in

220,0 g Tetrahydrofuran,

140,0 g Ethylenglykolmonomethylether und

44,0 g Butylacetat,

wird auf eine 0,3 mm starke, elektrochemisch aufgerauhte und mit Polyvinylphosphonsäure nachbehandelte Aluminiumfolie mittels einer Rakel derart aufgetragen, daß nach dem Verdunsten des Lösungsmittelgemischs eine photoleitfähige Schicht mit einem Gewicht von 5,2 $g/m^2$ zurückbleibt.

Die Schicht wird mit einer Corona auf -450 V aufgeladen und in einer Reprokamera mit 10 Halogenstrahlern zu je 600 W 14 sec lang belichtet. Als Vorlage dient eine Klebemontage, die die üblichen Prüfelemente enthält.

Nach der Entwicklung des durch Belichtung entstandenen latenten Ladungsbildes mit einem handelsüblichen Trockenentwickler und dessen thermischer Fixierung erhält man ein sauberes, grundfreies und randscharfes Bild der Vorlage.

Zur Umwandlung in eine Druckform bringt man die Aluminiumfolie mit der das fixerte Tonerbild enthaltenden Photoleiterschicht in eine Küvette, die eine Entschichterlösung enthält. Die Entschichterlösung wurde dadurch erhalten, daß man 50 g Natriumsilikat in 250 g Glycerin (86 %-ig) löste und mit 390 g Ethylenglykol und 310 g Methanol verdünnte.

Die ausentwickelte Druckplatte bringt in einer Bogenoffsetdruckmaschine einige zehntausend hervorragende Drucke.

**Beispiel 2**

Ein wie in Beispiel 1 hergestelltes elektrophotographisches Aufzeichnungsmaterial wird in einem Dyntest-Gerät vermessen. Die in diesem Gerät verwendete Glühfadenlampe hat eine Temperatur von 2800° K. Die Schicht wird auf -500 V aufgeladen und die Dunkelentladung aufgezeichnet. Nach 1 min beträgt das Restpotential $U_D$ -402 V 80,4 %.

Wird die Schicht auf -500 V aufgeladen und belichtet, so wird nach 1 min ein Restpotential $U_H$ von -3 V = 0,6 % erhalten. Die Schicht ist nach 12 sec auf $E_1/10$ = -50 V entladen. Die Halbwertsempfindlichkeit $E_{1/2}$ beträgt 11,5 $uJ/cm^2$.

**Vergleichsbeispiele**

In den nachfolgenden Beispielen wird die Schichtformulierung des Beispiels 1 verwendet, wobei das erfindungsgemäße Chinoxalin des Beispiels 1 durch die in Formeltabelle 2 aufgeführten Verbindungen ersetzt wird. Das Schichtgewicht der erhaltenen Schichten liegt zwischen 5,0 und 5,3 $g/m^2$. Die erhaltenen Schichten werden analog zu Beispiel 2 vermessen.

A. Photoleiter: 2- (4'-Dimethylaminophenyl)-3- (4'-chlorphenyl)-chinoxalin (Verbindung V)
Aufladung: -500 V
Restpotential $U_D$ nach 1 min: -418 V = 83,6 %
Restpotential $U_H$ nach 1 min: - 85 V = 17,0 %
Halbwertsempfindlichkeit $E_{1/2}$ = 33,6 $uJ/cm^2$

B. Photoleiter: 2(4'-Dimethylaminophenyl)-3-phenyl-6-methory-chinoxalin (Verbindung VI)
Aufladung: -500 V
Restpotential $U_D$ nach 1 min: -405 V = 81,0 %
Restpotential $U_H$ nach 1 min: - 71 V = 14,0 %
Halbwertsempfindlichkeit $E_{1/2}$ = 30,0 $uJ/cm^2$

C. Photoleiter: 2- (4' -Dimethylaminophenyl) -3-phenyl-chinoxalin (Verbindung VII)
Aufladung: -500 V
Restpotential $U_D$ nach 1 min: -458 V = 91,6 %
Restpotential $U_H$ nach 1 min: - 68 V = 13,5 %
Halbwertsempfindlichkeit $E_{1/2}$ = 26,6 $uJ/cm^2$

D. Photoleiter: 2-(4'-Dimethylaminophenyl)-3-phenyl-6-methyl-chinoxalin (Verbindung VIII)
Aufladung: -500 V
Restpotential $U_D$ nach 1 min: -463 V = 92,5 %
Restpotential $U_H$ nach 1 min: - 61 V = 12,2 %
Halbwertsempfindlichkeit $E_{1/2}$ = 32,6 $uJ/cm^2$

**Beispiel 3**

Wie in Beispiel 1 beschrieben wird eine Beschichtungslösung hergestellt, wobei der Photoleiter des Beispiels 1 durch die Verbindung 2,3-Bis-(4'-dimethylaminophenyl)-6-methyl-chinoxalin (Verbindung II) ersetzt wird Die Lösung wird mittels einer Rakel aui eine elektrochemisch aufgerauhte und mit Polyvinylphosphonsäure nachbehandelte Aluminiumfolie aufgetragen, wobei man ein Trockenschichtgewicht von 5,9 $g/m^2$ erhält. Das Material wird wie in Beispiel 2 beschrieben vermessen.

Aufladung: -500 V
Restpotential $U_D$ nach 1 min: -445 V = 89,0 %
Restpotential $U_H$ nach 1 min: - 11 V = 2,2 %
Halbwertsempfindlichkeit $E_{1/2}$ = 13,5 uJ/$cm^2$

**Beispiel 4**

Der Photoleiter des Beispiels 3 wird durch 2,3-Bis(4'-diethylaminophenyl)-6-methyl-chinoxalin (Verbindung I) ersetzt. Bei einem Trockenschichtgewicht von 5,4 g/$m^2$ erhält man folgende Meßwerte:
Aufladung: -500 V
Restpotential $U_D$ nach 1 min: -443 V = 88,6 %
Restpotential $U_H$ nach 1 min: - 5 V = 1,0 %
Halbwertsempfindlichkeit $E_{1/2}$ = 10,8 uJ/$cm^2$

**Beispiel 5**

Eine Beschichtungslösung, bestehend aus
30,0 g eines Umsetzungsprodukts aus einem Polyvinylbutyral des mittleren Molekulargewichts 80.000 mit 71 Gewichtsprozent Vinylbutyral-, 27 Gewichtsprozent Vinylalkohol- und 2 Gewichtsprozent Vinylacetatgruppen und Propenylsulfonylisocyanat mit der Säurezahl 158,
23,0 g 2,3-Bis(4'-diethylaminophenyl)-6-methyl-chinolaxin (Verbindung I),
0,1 g Rhodamin FB (C.I. 45170),
0,6 g Astrazonorange R (C.I. 48040) in
220,0 g Tetrahydrofuran,
140,0 g Ethylenglykolmonomethylether und
44,0 g Butylacetat,
wird wie in Beispiel 1 beschrieben mit einem Trockenschichtgewicht von 6,0 g/$m^2$ auf eine Aluminiumfolie aufgetragen. Die entsprechend Beispiel 2 vermessene Schicht ergibt folgende Meßwerte:
Aufladung: -500 V
Restpotential $U_D$ nach 1 min: -407 V = 81,4 %
Restpotential $U_H$ nach 1 min: - 5 V = 1,0 %
Halbwertsempfindlichkeit $E_{1/2}$ = 12,0 uJ/$cm^2$

**Beispiel 6**

Eine Beschichtungslösung, bestehend aus
25,0 g eines Copolymerisats aus Styrol und Maleinsäureanhydrid,
17,5 g 2,3-Bis(4'-diethylaminophenyl)-6-methylchinoalin (Verbindung I),
0,4 g Astrazonorange R (C.I. 48040),
0,2 g Brillantgrün (C.I. 42040) in
125,0 g Tetrahydrofuran,
80,0 g Ethylenglykolmonomethylether und
25,0 g Butylacetat,
wird auf eine elektrochemisch aufgerauhte und mit Polyvinylphosphonsäure nachbehandelte Aluminiumfolie aufgetragen und zu einem Schichtgewicht von 6,1 g/$m^2$ getrocknet.
Entsprechend Beispiel 2 erhält man für diese Schicht folgende Meßwerte:
Aufladung: -500 V
Restpotential $U_D$ nach 1 min: -355 V = 71,0 %
Restpotential $U_H$ nach 1 min: - 2 V = 0,4 %
Halbwertsempfindlichkeit $E_{1/2}$ = 7,5 uJ/$cm^2$

**Beispiel 7**

Die in Beispiel 1 beschriebene Beschichtungslösung wird derart variiert, daß 42,4 g des Photoleiters (Verbindung III) und 10,6 g des Copolymerisats eingesetzt werden. Man erhält eine elektrophotographische Schicht mit einem Trockengewicht von 6,5 g/$m^2$. Der Photoleiter kristallisiert nicht aus. Man erhält entsprechend Beispiel 2 folgende Meßwerte:

Aufladung: -500 V
Restpotential $U_D$ nach 1 min: -445 V = 89,0 %
Restpotential $U_H$ nach 1 min: praktisch Null
Halbwertsempfindlichkeit $E_{1/2}$ = 8,0 uJ/$cm^2$

**Beispiel 8**

Herstellung von 2,3-Bis(4'-dimethylaminophenyl)-6,7-dimethyl-chinoxalin (Verbindung IV)

14,8 g 4,4'-Bisdimethylaminobenzil und 7,5 g 4,5-Dimethyl-o-phenylendiamin werden in 100 ml Essigsäure 3 h am Rückfluß erhitzt. Die dunkelfarbene Lösung läßt man erkalten und gießt sie dann in Eiswasser. Die entstandene Mischung wird gut gerührt und der gelbe Niederschlag abgesaugt. Nach Trocknung wird aus Ethanol umkristallisiert.

Ausbeute: 18,2 g = 92 % d. Th., intensiv gelbe Nadeln
Festpunkt: 196 bis 197° C

Tabelle 1

I

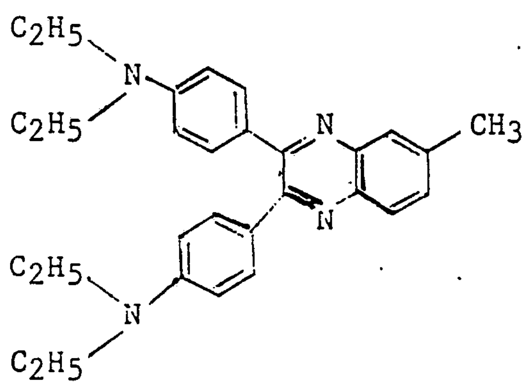

II

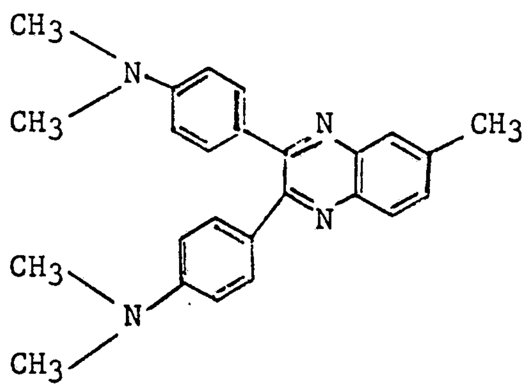

III

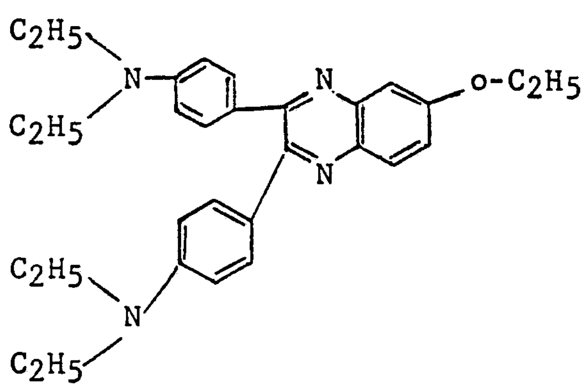

Fortsetzung Tabelle 1

IV

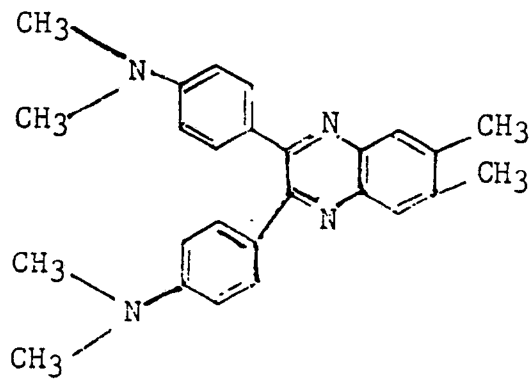

Tabelle 2

V

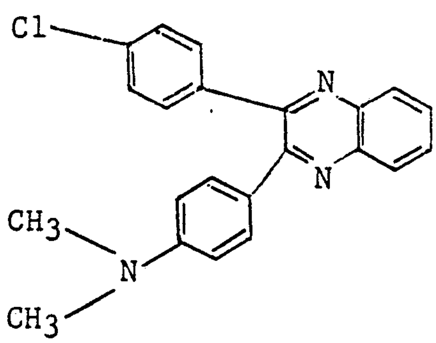

VI

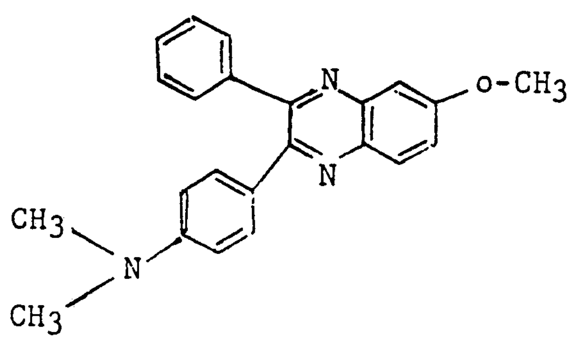

Fortsetzung Tabelle 2

VII

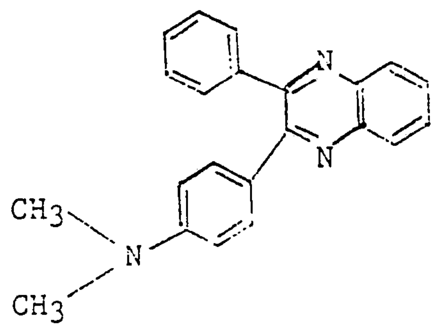

VIII

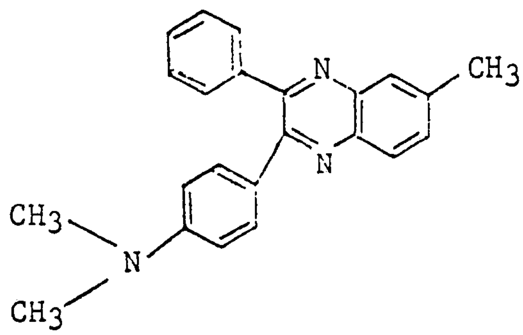

**Patentansprüche**

1. 2,3-Bis(dialkylaminophenyl) chinoxaline der allgemeinen Formel

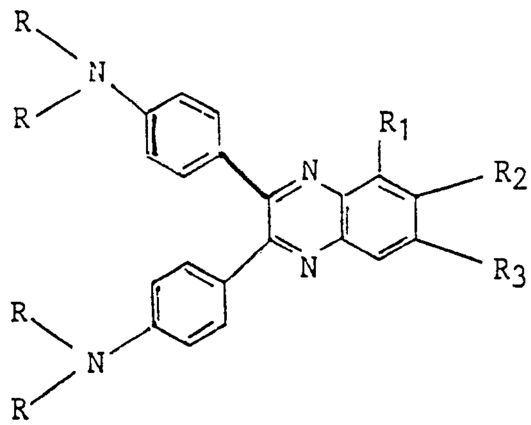

in welcher R Alkyl mit bis zu 4 Kohlenstoffatomen,
$R_1$ Wasserstoff oder Alkyl mit bis zu 4 Kohlenstoffatomen,
$R_2$ gleich oder verschieden ist von $R_1$ und Alkyl oder Alkoxy mit bis zu 4 Kohlenstoffatomen und
$R_3$ gleich oder verschieden ist von $R_2$ und Wasserstoff, Alkyl oder Alkoxy mit bis zu 4 Kohlenstoffatomen bedeuten.

2. 2,3-Bis(dialkylaminophenyl) chinoxaline nach Anspruch 1, dadurch gekennzeichnet, daß
R Methyl oder Ethyl,
$R_1$ Wasserstoff oder Methyl,
$R_2$ Methyl, Ethyl, Methoxy oder Ethoxy und
$R_3$ Wasserstoff, Methyl oder Methoxy bedeuten.

3. 2,3-Bis(dialkylaminophenyl) chinoxaline nach Anspruch 1 oder 2, dadurch gekennzeichnet, daß $R_1$, $R_2$ und $R_3$ gleich oder verschieden sind und Wasserstoff, Methyl, Methoxy oder Ethoxy bedeuten, wobei höchstens zwei der Reste $R_1$, $R_2$ und $R_3$ gleichzeitig Wasserstoff, Methyl, Methoxy oder Ethoxy darstellen.

4. 2,3-Bis(dialkylaminophenyl) chinoxaline nach Ansprüchen 1 bis 3, dadurch gekennzeichnet, daß sie 2,3-Bis(4'-diethylaminophenyl)-6-methyl-chinoxalin, 2,3-Bis(4'-dimethylaminophenyl)-6-methyl-chinoxalin, 2,3-Bis(4'-diethylaminophenyl)-6-ethoxy-chinoxalin oder 2,3-Bis(4'-dimethylaminophenyl) 6,7-dimethyl-chinoxalin darstellen.

5. Verwendung der 2,3-Bis(dialkylaminophenyl) chinoxaline nach Ansprüchen 1 bis 4 als photoleitfähige Substanzen in elektrophotographischem Aufzeichnungsmaterial.

6. Verwendung der 2,3-Bis(dialkylaminophenyl) chinoxaline nach Anspruch 5 zusammen mit alkalilöslichen Bindemitteln und Sensibilisierungsfarbstoffen mit einem Photoleiteranteil zwischen 20 und 95 Gewichtsprozent, bezogen auf die Photoleiterschicht.

7. Verwendung der 2,3-Bis(dialkylaminophenyl) chinoxaline nach Anspruch 5 oder 6 im Gemisch mit mindestens einem weiteren organischen oder anorganischen Photoleiter.

## Claims

1. 2,3-Bis(dialkylaminophenyl)quinoxalines of the general formula

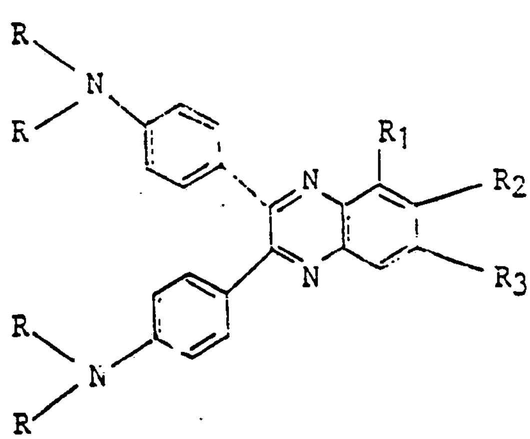

in which
R denotes an alkyl group up to 4 carbon atoms
$R_1$ denotes hydrogen or an alkyl group having up to 4 carbon atoms,

$R_2$ is identical with or different from $R_2$ and denotes an alkyl or alkoxy group having up to 4 carbon atoms, and

$R_3$ is identical with or different from $R_2$ and denotes hydrogen, an alkyl or an alkoxy group having up to 4 carbon atoms.

2. 2,3-Bis(dialkylaminophenyl)quinoxalines as claimed in Claim 1, wherein

R denotes a methyl or ethyl group,

$R_1$ denotes hydrogen or a methyl group,

$R_2$ denotes a methyl, ethyl, methoxy or ethoxy group and

$R_3$ denotes hydrogen, a methyl or a methoxy group.

3. 2,3-Bis(dialkylaminophenyl)quinoxalines as claimed in Claim 1 or 2, wherein $R_1$, $R_2$ and $R_3$ are identical or different and denote hydrogen, a methyl, a methoxy or an ethoxy group, whereby not more than two of the groups $R_1$, $R_2$ and $R_3$ simultaneously denote hydrogen, a methyl, a methoxy or an ethoxy group.

4. 2,3-Bis(dialkylaminophenyl)quinoxalines as claimed in Claims 1 to 3, which are 2,3-bis(4-diethylaminophenyl)-6-methyl quinoxaline, 2,3-bis(4'-dimethylami-nophenyl)-6-methyl quinoxaline, 2,3-bis(4'-dimethylaminophenyl)-6-ethoxy quinoxaline or 2,3-bis(4'-dimethylaminophenyl)-6,7dimethyl quinoxaline.

5. Use of the 2,3-bis(dialkylaminophenyl)quinoxalines as claimed in any of Claim 1 to 4 as photoconductive substances in electrophotographic recording materials.

6. Use of the 2,3-bis(dialkylaminophenyl)quinoxalines as claimed in Claim 5 together with alkali-soluble binders and sensitizing dye-stuffs, whereby the amount of photoconductor is between 20 and 95 % by weight, relative to the photoconductive layer.

7. Use of the 2,3-bis(dialkylaminophenyl)quinoxalines, as claimed in Claim 5 or 6, in a mixture with at least one further organic or inorganic photoconductor.

**Revendications**

1. 2,3-bis(dialkylaminophényl)quinoxalines de formule générale

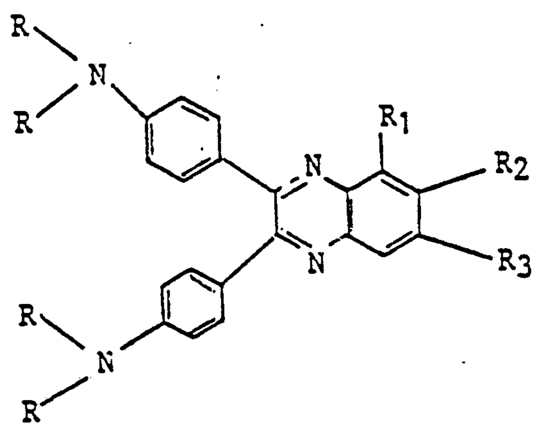

dans laquelle

R représente un groupe alkyle ayant jusqu'à 4 atomes de carbone,

$R_1$ représente un atome d'hydrogène ou un groupe alkyle ayant jusqu'à 4 atomes de carbone,

$R_2$ est identiqué à $R_1$ ou différent de celui-ci, et représente un groupe alkyle ou alcoxy ayant jusqu'à 4 atomes de carbone, et

$R_3$ est identique à $R_2$ ou différent de celui-ci, et représente un atome d'hydrogène, un groupe alkyle ou un groupe alcoxy ayant jusqu'à 4 atomes de carbone.

2. 2,3-bis(dialkylaminophényl)quinoxalines selon la revendication 1, caractérisées en ce que

R représente le groupe méthyle ou éthyle,

$R_1$ représente un atome d'hydrogène ou le groupe méthyle,

$R_1$ représente le groupe méthyle, éthyle, méthoxy ou éthoxy, et

$R_3$ représente un atome d'hydrogène ou le groupe méthyle ou méthoxy.

3. 2,3-bis(dialkylaminophényl)quinoxalines selon la revendication 1 ou 2, caractérisées en ce que $R_1$ , $R_2$ et $R_3$ sont identiques ou différents et représentent un atome d'hydrogène, le groupe méthyle, méthoxy ou éthoxy, deux au plus des radicaux $R_1$, $R_2$ et $R_3$ représentant simultanément des atomes d'hydrogène ou les groupes méthyle, méthoxy ou éthoxy.

4. 2,3-bis(dialkylaminophényl)quinoxalines selon les revendications 1 à 3, caractérisées en ce qu'elles sont constituées par la 2,3-bis(4'-diéthylaminopnényl)-6-méthyl-quinoxaline, la 2,3-bis(4'-diméthylaminophényl)-6-méthylquinoxaline, la 2,3-bis(4'-diéthylaminophényl)-6-éthoxy-quinoxaline ou la 2,3-bis(4'-diméthylaminophényl)-6,7-diméthyl-quinoxaline.

5. Utilisation des 2,3-bis(dialkylaminophényl)-quinoxalines selon les revendications 1 à 4, en tant que

substances photoconductrices dans un matériau de reproduction électrophotographique.

6. Utilisation des 2,3-bis(dialkylaminophényl)-quinoxalines selon la revendication 5, conjointement avec des liants solubles dans des alcalis et des colorants sensibilisateurs, la proportion du photoconducteur étant comprise entre 20 et 95 % en poids, par rapport à la couche photoconductrice.

7. Utilisation des 2,3-bis(dialkylaminophényl)-quinoxalines selon la revendication 5 ou 6, en mélange avec au moins un autre photoconducteur organique ou inorganique.